# FASCICULE DE BREVET EUROPEEN

(11) **EP 0 650 959 B1**
(45) Date de publication et mention de la délivrance du brevet: **03.09.1997**
(21) Numéro de dépôt: 94420288.6
(22) Date de dépôt: 24.10.1994
(51) Int. Cl.: C07C 253/10

(54) **Procédé d'hydrocyanation de nitriles insaturés en dinitriles**
Hydrocyanierung von ungesättigten Nitrilen zu Dinitrilen
Hydrocyanation process of unsatured nitriles into dinitriles

(30) Priorité: 03.11.1993 FR 9313306
(43) Date de publication de la demande: 03.05.1995
(73) Titulaire: RHONE-POULENC FIBER & RESIN INTERMEDIATES, 92405 Courbevoie (FR)
(72) Inventeur: Huser, Marc, F-69100 Villeurbanne (FR); Perron, Robert, F-69390 Charly (FR)
(74) Mandataire: Vignally, Noel

(56) Documents cités:
- FR-A- 2 338 253
- US-A- 3 496 217
- US-A- 4 705 881

## Description

La présente invention concerne un procédé d'hydrocyanation de nitriles à insaturation éthylénique en dinitriles saturés, notamment l'hydrocyanation des pentène-nitriles en adiponitrile, l'un des composés de base pour la production du polyamide 66.

Le brevet français n° 1 599 761 décrit un procédé de préparation de nitriles par addition d'acide cyanhydrique sur des composés organiques ayant au moins une double liaison éthylénique, en présence d'un catalyseur au nickel et d'un phosphite de triaryle. Cette réaction peut être conduite en présence ou non d'un solvant.

Lorsqu'un solvant est utilisé dans ce procédé de l'art antérieur, il s'agit de préférence d'un hydrocarbure, tel que le benzène ou les xylènes ou d'un nitrile tel que l'acétonitrile.

Le catalyseur mis en oeuvre est un complexe organique de nickel, contenant des ligands tels que les phosphines, les arsines, les stilbines, les phosphites, les arsénites ou les antimonites.

La présence d'un promoteur pour activer le catalyseur, tel qu'un composé du bore ou un sel métallique, généralement un acide de Lewis, est également préconisée dans ledit brevet.

Dans ce procédé, le milieu est entièrement organique et l'un de ses inconvénients majeurs réside dans la difficulté de séparer, en fin de réaction, les produits de l'hydrocyanation, de la solution catalytique contenant plusieurs constituants (complexe du nickel, phosphite de triaryle, promoteur), en vue notamment du recyclage de cette dernière dans une nouvelle réaction d'hydrocyanation. Une telle séparation est délicate et imparfaite et on constate une perte substantielle de catalyseur, ainsi que la présence dudit catalyseur dans les produits d'hydrocyanation.

Le brevet FR-A-2 338 253 a proposé de réaliser l'hydrocyanation des composés ayant au moins une insaturation éthylénique, en présence d'une solution aqueuse d'un composé d'un métal de transition, notamment le nickel, le palladium ou le fer, et d'une phosphine sulfonée.

Ce dernier procédé permet une bonne hydrocyanation, des pentène-nitriles notamment, et une séparation aisée de la solution catalytique par simple décantation.

Les résultats obtenus lors de la réaction d'hydrocyanation sont relativement bons avec les différents substrats et notamment avec les oléfines fonctionnalisées comme les pentène-nitriles. Il s'avère cependant que le pourcentage de composé dinitrile linéaire formé par rapport à l'ensemble des isomères obtenus ne dépasse généralement pas 65 % à 70 %.

En outre, on constate que le catalyseur se désactive rapidement.

La présente invention se propose de pallier ces inconvénients, en particulier d'améliorer le taux de linéarité des produits formés et/ou d'augmenter la durée de vie du catalyseur.

Elle consiste en un procédé d'hydrocyanation d'un nitrile aliphatique à insaturation éthylénique, par réaction avec le cyanure d'hydrogène, en présence d'une solution aqueuse d'un catalyseur comprenant un composé d'un métal de transition et une phosphine sulfonée, caractérisé en ce que ladite solution contient également un cocatalyseur consistant en au moins un acide de Lewis.

Les nitriles à insaturation éthylénique plus particulièrement mis en oeuvre dans le présent procédé sont les pentène-nitriles linéaires comme le pentène-3 nitrile, le pentène-4 nitrile et leurs mélanges.

Ces pentène-nitriles peuvent contenir des quantités, généralement minoritaires, d'autres composés, comme le méthyl-2 butène-3 nitrile, le méthyl-2 butène-2 nitrile, le pentène-2 nitrile, le valéronitrile, l'adiponitrile, le méthyl-2 glutaronitrile, l'éthyl-2 succinonitrile ou le butadiène, provenant de la réaction antérieure d'hydrocyanation du butadiène et/ou de l'isomérisation du méthyl-2 butène-3 nitrile en pentène-nitriles.

On utilise de préférence comme composés des métaux de transition des composés du nickel, du palladium et du fer. On utilise des composés solubles dans l'eau ou capables de passer en solution dans les conditions de la réaction. Le reste lié au métal n'est pas critique, dès l'instant qu'il satisfait à ces conditions.

Parmi les composés précités, les composés les plus préférés sont ceux du nickel, on peut citer à titre d'exemples non limitatifs :
- les composés dans lesquel le nickel est au degré d'oxydation zéro comme le tétracyanonickelate de potassium K₄ (NiCN₄), le bis (acrylonitrile) nickel zéro, le bis (cyclooctadiène-1,5)₂ nickel et les dérivés contenant des ligands du groupe Va comme le tétrakis (triphényl-phosphine) nickel zéro (dans ce dernier cas le composé peut être dissous dans un solvant non miscible à l'eau comme le toluène, puis une solution aqueuse de phosphine sulfonée extrait une partie du nickel en développant une coloration rouge dans la solution aqueuse qui décante);
- les composés du nickel comme les carboxylates (notamment l'acétate), carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkyl-sulfonates.

Il n'est pas nécessaire que le composé du nickel soit lui même soluble dans l'eau. Par exemple, le cyanure de nickel peu soluble dans l'eau se dissout très bien dans une solution aqueuse de phosphine.

Quand le composé du nickel utilisé correspond à un état d'oxydation du nickel supérieur à 0, on ajoute au milieu réactionnel un réducteur du nickel réagissant préférentiellement avec le nickel dans les conditions de la réaction. Ce réducteur peut être organique ou minéral. On peut citer comme exemples non limitatifs le BH₄Na, la poudre de Zn, le magnésium, le BH₄K et les borohydrures de préférence solubles dans l'eau.

Ce réducteur est ajouté en quantité telle que le nombre d'équivalents d'oxydo-réduction est compris entre 1 et 10. Des valeurs inférieures à 1 et supérieures à 10 ne sont toutefois pas exclues.

Lorsque le composé du nickel utilisé correspond à l'état d'oxydation 0 du nickel, on peut également ajouter un réducteur du type de ceux précités, mais cet ajout n'est pas impératif.

Lorsqu'on utilise un composé du fer, les mêmes réducteurs conviennent.

Dans le cas du palladium, les réducteurs peuvent être, en outre, des éléments du milieu réactionnel (phosphine, solvant, oléfine).

Les phosphines sulfonées mises en oeuvre dans le présent procédé sont plus particulièrement les phosphines sulfonées de formule générale (I) : 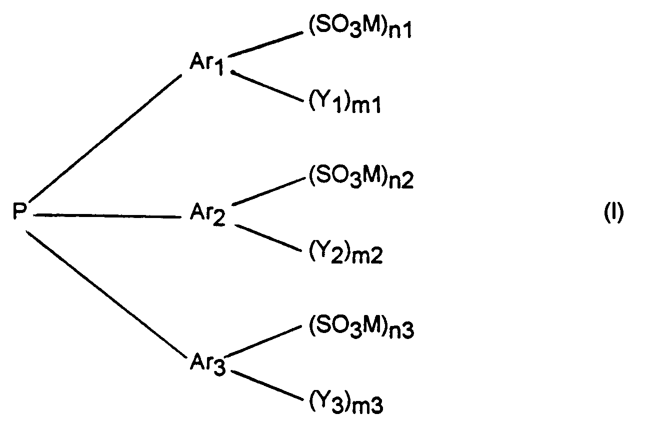 dans laquelle :
* Ar₁, Ar₂ et Ar₃, identiques ou différents, représentent des groupements aryles
* Y₁, Y₂ et Y₃, identiques ou différents, représentent
   . un radical alkyle ayant 1 à 4 atomes de carbone,
   . un radical alcoxy ayant 1 à 4 atomes de carbone,
   . un atome d'halogène,
   . un radical CN,
   . un radical NO₂
   . un radical OH,
   . un radical NR₁R₂, dans la formule duquel R₁ et R₂, identiques ou différents, représentent un radical alkyle ayant 1 à 4 atomes de carbone,
* M est un reste cationique minéral ou organique choisi, de manière à ce que le composé de formule (I) soit soluble dans l'eau, dans le groupe comprenant :
   . H⁺,
   . les cations dérivés des métaux alcalins ou alcalino-terreux,
   . N(R₃R₄R₅R₆)⁺ avec R₃,R₄,R₅ et R₆, identiques ou différents, représentant un radical alkyle ayant 1 à 4 atomes de carbone ou un atome d'hydrogène,
   . les autres cations dérivés des métaux dont les sels de l'acide benzènesulfonique sont solubles dans l'eau,
* m₁, m₂ et m₃ sont des nombres entiers, identiques ou différents, de 0 à 5,
* n₁, n₂ et n₃ sont des nombres entiers, identiques ou différents, de 0 à 3, l'un d'entre eux au moins étant égal ou supérieur à 1.

Comme exemples de métaux dont les sels de l'acide benzènesulfonique sont solubles dans l'eau, on peut citer le plomb, le zinc et l'étain.

Par l'expression soluble dans l'eau, on entend dans le présent texte un composé soluble à au moins 0,01 g par litre d'eau.

Parmi les phosphines de formule (I), on préfère celles pour lesquelles :
- Ar₁, Ar₂ et Ar₃ sont des groupements phényle,
- Y₁, Y₂ et Y₃ représentent des groupements choisis parmi
   . les radicaux alkyle ayant de 1 à 2 atomes de carbone,
   . les radicaux alkoxy ayant de 1 à 2 atomes de C - C₁,
- M représente un cation choisi parmi le groupe comprenant
   . H⁺
   . les cations dérivés de Na, K, Ca, Ba
   . NH₄+
   . les cations tétraméthylammonium, tétraéthylammonium, tétrapropylammonium, tétrabutylammonium
- m₁, m₂ et m₃ sont des nombres entiers de 0 à 3
- n₁, n₂ et n₃ sont des nombres entiers de 0 à 3, l'un au moins étant égal ou supérieur à 1.

Parmi ces phosphines, les plus particulièrement préférées sont les sels de sodium, de potassium, de calcium, de baryum, d'ammonium, de tétraméthylammonium et de tétraéthylammonium, des mono(sulfophényl) diphényl-phosphine, di(sulfophényl) phényl-phosphine et tri(sulfophényl)-phosphine dans les formules desquelles les groupements SO₃ sont de préférence en position méta.

On peut citer comme autres exemples de phosphines de formule (I) pouvant être utilisées selon le procédé de l'invention, les sels alcalins ou alcalino-terreux, les sels d'ammonium, les sels d'ammonium quaternaire des (sulfo-3 méthyl-4 phényl) di (méthyl-4 phényl)-phosphine; (sulfo-3 méthoxy-4 phényl) di(méthoxy-4 phényl)-phosphine ; (sulfo-3 chloro-4 phényl) di(chloro-4 phényl)-phosphine ; di(sulfo-3 phényl) phényl-phosphine ; di(sulfo-4 phényl) phényl-phosphine ; di(sulfo-3 méthyl-4 phényl) (méthyl-4 phényl)-phosphine ; di(sulfo-3 méthoxy-4 phényl) (méthoxy-4 phényl) phosphine ; di(sulfo-3 chloro-4 phényl) (chloro-4 phényl) phosphine ; tri(sulfo-3 phényl)-phosphine ; tri(sulfo-4 phényl)-phosphine ; (tri(sulfo--3 méthyl-4 phényl)-phosphine ; tri(sulfo-3, méthoxy-4 phényl)-phosphine ; tri(sulfo-3 chloro-4 phényl)-phosphine ; (sulfo-2 méthyl-4 phényl) (sulfo-3, méthyl-4 phényl) (disulfo-3,5 méthyl-4 phényl)-phosphine ; (sulfo-3 phényl) (sulfo-3 chloro-4 phényl) (disulfo-3,5 chloro-4 phényl)-phosphine.

On peut bien évidemment utiliser un mélange de ces phosphines. En particulier, on peut utiliser un mélange de phosphines mono-, di- et tri-métasulfonées.

Les phosphines sulfonées utilisées dans le procédé selon la présente invention peuvent être préparées en appliquant les procédés connus. Ainsi, conformément à l'enseignement de H. SCHINDLBAUER, Monatsch. Chem. 96, pages 2051-2057 (1965), on peut préparer le sel de sodium de la (p-sulfophényl)diphényl phosphine en faisant réagir, en présence de sodium ou de potassium,
du p-chlorobenzène-sulfonate de sodium avec la diphénylchlorophosphine. Selon la méthode décrite dans J. Chem. Soc., pages 276-288 (1958) et dans le brevet anglais n° 1 066 261, on peut préparer des phénylphosphines de formule (I) en faisant appel à la réaction de sulfonation de noyaux aromatiques à l'aide d'oléum, puis en procédant à la neutralisation des groupes sulfoniques formés au moyen d'un dérivé basique approprié d'un des métaux que représente M dans la formule (I). La phosphine sulfonée brute obtenue peut contenir en mélange l'oxyde de phosphine sulfonée correspondant, dont la présence n'est cependant pas gênante pour exécuter le procédé d'hydrocyanation selon la présente invention.

La réaction d'hydrocyanation est généralement réalisée à une température de 10°C à 150°C et de préférence de 30°C à 120°C.

La quantité de composé du nickel utilisée est choisie de telle sorte qu'il y ait par litre de solution réactionnelle entre 10⁻⁴ et 1, et de préférence entre 0,005 et 0,5 mole de nickel.

La quantité de phosphine de formule (I) utilisée pour préparer la solution réactionnelle est choisie de telle sorte que le nombre de moles de ce composé rapporté à 1 mole de métal élémentaire soit de 0,5 à 2000 et de préférence de 2 à 300.

L'acide de Lewis utilisé comme cocatalyseur permet d'améliorer la linéarité des dinitriles obtenus, c'est-à-dire le pourcentage de dinitrile linéaire par rapport à la totalité des dinitriles formés, et/ou d'augmenter la durée de vie du catalyseur.

Par acide de Lewis, on entend dans le présent texte, selon la définition usuelle, des composés accepteurs de doublets électroniques.

On peut mettre en oeuvre notamment les acides de Lewis cités dans l'ouvrage édité par G.A. OLAH "Friedel-Crafts and related Reactions", tome I, pages 191 à 197 (1963).

Les acides de Lewis qui peuvent être mis en oeuvre comme cocatalyseurs dans le présent procédé sont choisis parmi les composés des éléments des groupes Ib, IIb, IIIa, IIIb, IVa, IVb, Va, Vb, VIb, VIIb et VIII de la Classification périodique des éléments, dans la mesure où lesdits composés sont au moins partiellement solubles et stables dans l'eau ou plus généralement dans la phase aqueuse du mélange réactionnel. Ces composés sont le plus souvent, mais de manière non limitative, des sels, notamment des halogénures, de préférence chlorures et bromures, des sulfates, des nitrates, des sulfonates, notamment des trifluorométhanesulfonates, des carboxylates, des acétylacétonates, des tétrafluoroborates et des phosphates.

A titre d'exemples non limitatifs de tels acides de Lewis, on peut citer le chlorure de zinc, le bromure de zinc, l'iodure de zinc, le trifluorométhanesulfonate de zinc, I'acétate de zinc, le nitrate de zinc, le tétrafluoroborate de zinc, le chlorure de manganèse, le bromure de manganèse, le chlorure de nickel, le bromure de nickel, le cyanure de nickel, l'acétylacétonate de nickel, le chlorure de cadmium, le bromure de cadmium, le chlorure stanneux, le bromure stanneux, le sulfate stanneux, le tartrate stanneux, les chlorures, bromures, sulfates, nitrates, carboxylates ou trifluorométhanesulfonates des éléments des terres rares comme le lanthane, le cérium, le praséodyme, le néodyme, le samarium, l'europium, le gadolinium, le terbium, le dysprosium, l'holmium, l'erbium, le thulium, l'ytterbium et le lutétium, le chlorure de cobalt, le chlorure ferreux, le chlorure d'yttrium.

On peut bien entendu mettre en oeuvre des mélanges de plusieurs acides de Lewis.

Il est également intéressant le cas échéant de stabiliser l'acide de Lewis en solution aqueuse par l'adjonction d'un chlorure de métal alcalin comme le chlorure de lithium ou le chlorure de sodium notamment. Le rapport molaire chlorure de lithium ou de sodium/acide de Lewis varie de manière très large, par exemple de 0 à 100, le rapport particulier pouvant être ajusté selon la stabilité dans l'eau de l'acide de Lewis.

Parmi les acides de Lewis, on préfère tout particulièrement le chlorure de zinc, le bromure de zinc, le sulfate de zinc, le tétrafluoroborate de zinc, le chlorure stanneux, le bromure stanneux, le chlorure stanneux stabilisé par le chlorure de lithium, le chlorure stanneux stabilisé par le chlorure de sodium, les mélanges chlorure de zinc/chlorure stanneux, le chlorure de nickel, le bromure de nickel, I'acétylacétonate de nickel.

Le cocatalyseur acide de Lewis mis en oeuvre représente généralement de 0,01 à 50 moles par mole de composé de métal de transition, plus particulièrement de composé du nickel, et de préférence de 1 à 10 mole par mole.

La solution catalytique utilisée pour l'hydrocyanation selon l'invention peut être préparée avant son introduction dans la zone de réaction, par exemple par addition à la solution aqueuse de la phosphine de formule (I), de la quantité appropriée de composé du métal de transition choisi, de l'acide de Lewis et éventuellement du réducteur. Il est également possible de préparer la solution catalytique "in situ" par simple mélange de ces divers constituants.

Bien que la réaction soit conduite généralement sans tiers-solvant, il peut être avantageux de rajouter un solvant organique inerte, non miscible à l'eau, qui pourra être celui de l'extraction ultérieure.

A titre d'exemples de tels solvants, on peut citer les hydrocarbures aromatiques, aliphatiques ou cycloaliphatiques qui maintiennent à l'état biphasique le milieu réactionnel.

Ainsi dès la fin de la réaction, il est très aisé de séparer, d'une part, une phase aqueuse contenant la phosphine sulfonée de formule (I), le composé de métal de transition et l'acide de Lewis et d'autre part, une phase organique constituée des réactifs engagés dans la réaction des produits de la réaction et le cas échéant du solvant organique non miscible à l'eau.

Parmi les solvants organiques susceptibles d'être utilisés dans le procédé d'hydrocyanation, on peut citer le benzène, le toluène, les xylènes, l'hexane, le cyclohexane.

Le procédé de l'invention peut être mis en oeuvre de manière continue ou discontinue.

Le cyanure d'hydrogène mis en oeuvre peut être préparé à partir des cyanures métalliques, notamment le cyanure de sodium, ou des cyanhydrines.

Le cyanure d'hydrogène est introduit dans le réacteur sous forme gazeuse ou sous forme liquide. Il peut également être préalablement dissous dans un solvant organique.

Dans le cadre d'une mise en oeuvre discontinue, on peut en pratique charger dans un réacteur, préalablement purgé à l'aide d'un gaz inerte (tel qu'azote, argon), soit une solution aqueuse contenant la totalité ou une partie des divers constituants tels que la phosphine sulfonée, le composé de métal de transition, les éventuels réducteur et solvant, l'acide de Lewis, soit séparément lesdits constituants. Généralement le réacteur est alors porté à la température choisie, puis le pentène-nitrile est introduit. Le cyanure d'hydrogène est alors lui-même introduit, de préférence de manière continue et régulière.

Lorsque la réaction (dont on peut suivre l'évolution par dosage de prélèvements) est terminée, le mélange réactionnel est soutiré après refroidissement et les produits de la réaction sont isolés par décantation, éventuellement suivie d'une extraction de la couche aqueuse à l'aide d'un solvant approprié, tel que par exemple les solvants non miscibles à l'eau cités précédemment.

La solution catalytique aqueuse peut alors être recyclée dans une nouvelle réaction d'hydrocyanation.

Dans le cadre d'une mise en oeuvre du procédé en continu, seule la phase organique peut être soutirée, tandis que la phase catalytique aqueuse demeure dans le réacteur.

Les exemples qui suivent illustrent l'invention.

### EXEMPLE 1

### 1) Préparation de la solution catalytique Ni/TPPTS.

Dans un ballon en verre de 1 litre, muni d'un barreau aimanté et d'un réfrigérant ascendant, on charge 500 cm³ d'une solution de 300 mmol de sel de sodium de triphénylphosphine trisulfonée (TPPTS) dans l'eau; on dégaze cette solution. On introduit ensuite, sous agitation et sous courant d'argon, 20 g (73 mmol) de Ni(cyclooctadiène)₂, puis 350 cm³ d'ortho-xylène préalablement dégazé.

On chauffe à 45°C pendant 15 h. Après refroidissement, le système biphasique est décanté et la phase aqueuse fortement colorée en rouge est prélevée.

### 2) Hydrocyanation du pentène-3 nitrile.

Dans un réacteur en verre de 150cm³, agité à l'aide d'une turbine, on charge 34,8 cm³ d'une solution aqueuse de catalyseur Ni/TPPTS, contenant 5 mmol de Ni et 20 mmol de TPPTS. On chauffe sous agitation à 60°C; puis en maintenant cette température on injecte successivement :
- 3,2 cm³ d'une solution aqueuse contenant 20 mmol de chlorure de Zn
- 8 g (105 mmol) de pentène-3 nitrile (3PN).

On injecte ensuite du cyanure d'hydrogène à raison de 1,8 g/h (67mmol/h) pendant 2 h.

En fin d'essai, on refroidit le mélange réactionnel obtenu, on neutralise à l'aide d'une solution concentrée de soude l'éventuel excès de cyanure d'hydrogène injecté et on dose les différents constituants par chromatographie en phase gazeuse (CPG).

On obtient les résultats suivants :

| | |
|---|---|
| - taux de transformation (TT) du 3PN | 89 % |
| - rendement (RT) en adiponitrile (ADN) par rapport au 3PN transformé | 66 % |
| - RT en méthyl-2 glutaronitrile (MGN) par rapport au 3PN transformé | 26 % |
| - RT en éthyl-2 succinonitrile (ESN) par rapport au 3PN transformé | 5 % |
| - RT en valéronitrile (VN) par rapport au 3PN transformé | 3 % |
| - linéarité(*) | 68 % |
| - activité du catalyseur (**) | 20 |
| - productivité en ADN (rapportée au volume de la phase aqueuse) | 90 g/h.l |

| | |
|---|---|
| (*) ADN formé/ADN + MGN + ESN formés | |
| (**) nombre de moles de 3PN transformé par mole de Ni engagé | |

### EXEMPLES 2 à 5 et essai comparatif 1

On répète l'exemple 1, avec les charges et les conditions opératoires suivantes:
- - Ni :: 5 mmol
- - TPPTS :: 20 mmol
- - ZnCl₂ :: voir tableau 1
- - eau :: qsp 38 ml
- - 3PN :: 320 mmol
- - débit d'injection de HCN :: 67 mmol/h
- - température :: 65°C

Le tableau 1 rassemble les résultats obtenus ( RT en DN = rendement en ADN+MGN+ESN par rapport au 3PN transformé).

**Tableau 1**

| Essai | ZnCl₂ (en mmol) | Activité du catalyseur | RT % en DN | Linéarité |
|---|---|---|---|---|
| Exemple 2 | 1 | 5 | 88 | 73 |
| Exemple 3 | 5 | 14 | 97 | 74 |
| Exemple 4 | 20 | 30 | 97 | 63 |
| Exemple 5 | 40 | 30 | 97 | 57 |
| Essai comparatif | 0 | 3 | 76 | 62 |

### EXEMPLE 6

On répéte l'exemple 1, avec les charges et les conditions opératoires suivantes:
- - Ni :: 10 mmol
- - TPPTS :: 20 mmol
- - ZnCl₂ :: 20 mmol
- - eau :: qsp 38 ml
- - 3PN :: 320 mmol
- - débit d'injection de HCN :: 67 mmol/h
- - température :: 65°C

On obtient les résutats suivants :
- - RT en DN :: 98 %
- - activité du catalyseur :: 15
- - linéarité :: 71 %

### EXEMPLES 7 et 8

On répète l'exemple 1, avec les charges et les conditions opératoires suivantes:
- - Ni :: 5 mmol
- - TPPTS :: voir tableau 2
- - ZnCl₂ :: 20 mmol
- - eau :: qsp 38 ml
- - 3PN :: 320 mmol
- - débit d'injection de HCN :: 67 mmol/h
- - température :: 65°C

Le tableau 2 rassemble les résultats obtenus.

**Tableau 2**

| Exemple | TPPTS (en mmol) | Activité du catalyseur | RT % en DN | Linéarité |
|---|---|---|---|---|
| Exemple 7 | 6 | 6 | 98 | 60 |
| Exemple 8 | 32 | 17 | 98 | 73 |

### EXEMPLES 9 et 10

On répète l'exemple 1, avec les charges et les conditions opératoires suivantes:
- - Ni :: 5 mmol
- - TPPTS :: 20 mmol
- - ZnCl₂ :: 20 mmol
- - eau :: qsp 38 ml
- - 3PN :: 320 mmol
- - débit d'injection de HCN :: voir tableau 3
- - température :: voir tableau 3

Le tableau 3 rassemble les résultats obtenus (l'exemple 4 a été également reporté dans le tableau).

**Tableau 3**

| Exemples | Température | Débit d'injection de HCN | Activité du catalyseur | RT % en DN | Linéarité |
|---|---|---|---|---|---|
| Exemple 9 | 45°C | 17 mmol/h | 35 | 99 | 68 |
| Exemple 4 | 65°C | 67 mmol/h | 30 | 97 | 63 |
| Exemple 10 | 85°C | 135 mmol/h | 10 | 99 | 63 |

### EXEMPLES 11 à 23

On répète l'exemple 1, avec les charges et les conditions opératoires suivantes:
- - Ni :: 5 mmol
- - TPPTS :: 20 mmol
- - Acide de Lewis (nature indiquée dans tableau 4) :: 20 mmol
- - eau :: qsp 35 ml
- - 3PN :: 200 mmol
- - débit d'injection de HCN :: 67 mmol/h
- - température :: 65°C
- - durée :: 1 h

Le tableau 4 rassemble les résultats obtenus.

### EXEMPLES 24 à 49

On répète l'exemple 1, avec les charges et les conditions opératoires suivantes:
- - Ni :: 5 mmol
- - TPPTS :: 20 mmol
- - Acide de Lewis (nature indiquée dans tableau 5) :: 20 mmol
- - eau :: qsp 35 ml
- - 3PN :: 200 mmol
- - débit d'injection de HCN :: 67 mmol/h
- - température :: 65°C
- - durée :: 1 h

Le tableau 5 rassemble les résultats obtenus.

**Tableau 4**

| Exemple | Acide de Lewis | Activité du catalyseur | Linéarité |
|---|---|---|---|
| Exemple 11 | MnBr₂ | 8 | 64 |
| Exemple 12 | MnCl₂ | 8 | 66 |
| Exemple 13 | FeCl₂ | 6 | 68 |
| Exemple 14 | CoCl₂/ZnCl₂(*) | 19 | 72 |
| Exemple 15 | ZnBr₂ | 25 | 68 |
| Exemple 16 | Znl₂ | 23 | 66 |
| Exemple 17 | YCl₃ | 8 | 75 |
| Exemple 18 | CdBr₂ | 20 | 69 |
| Exemple 19 | SnBr₂ | 10 | 88 |
| Exemple 20 | SnCl₂ | 12 | 88 |
| Exemple 21 | SnCl₂/LiCl(**) | 15 | 87 |
| Exemple 22 | SnCl₂/ZnCl₂(*) | 15 | 83 |
| Exemple 23 | CeBr₃ | 6 | 64 |

| | | | |
|---|---|---|---|
| (*) mélange équimoléculaire de 2 acides de Lewis | | | |
| (**) addition supplémentaire de 200 mmol de LiCl | | | |

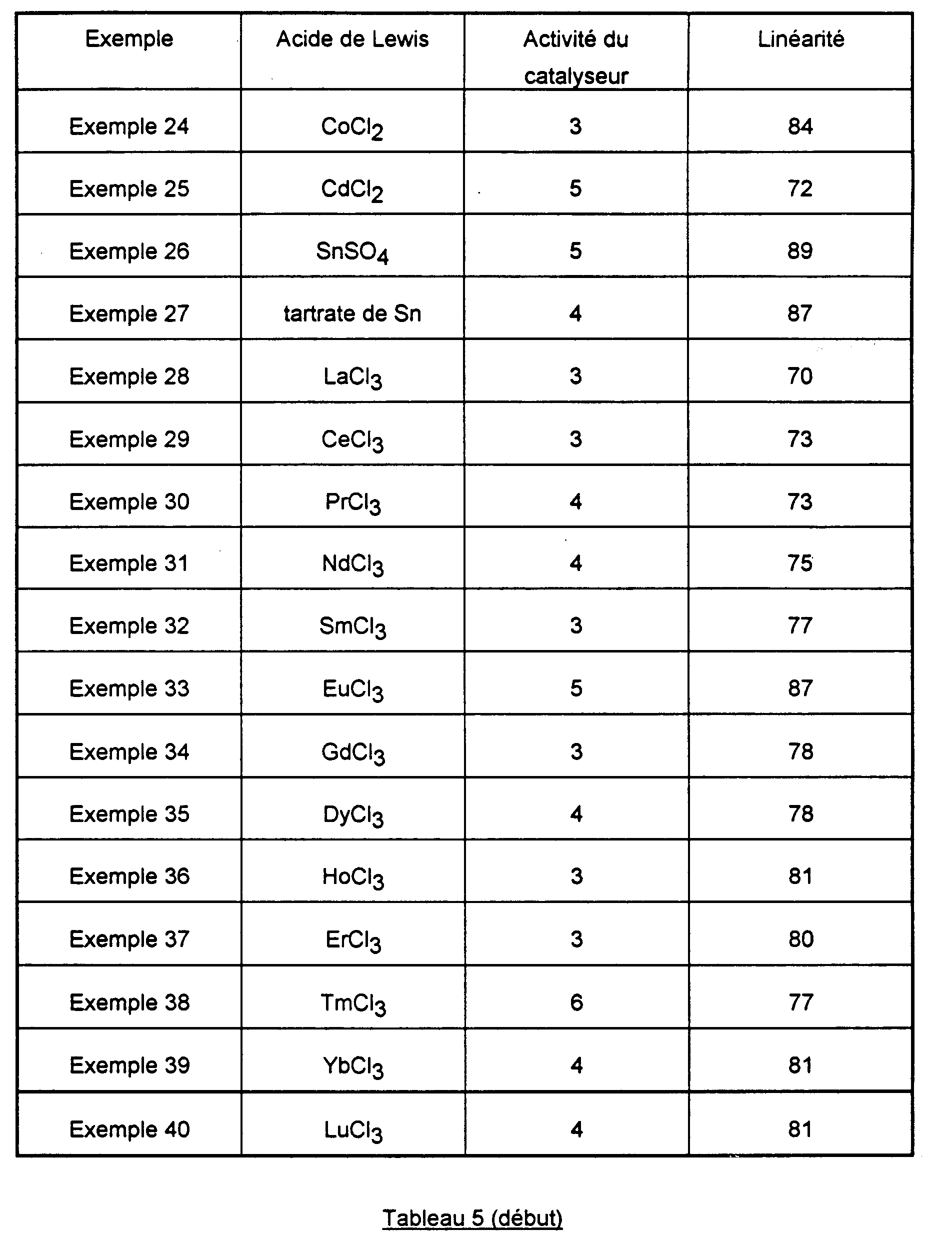

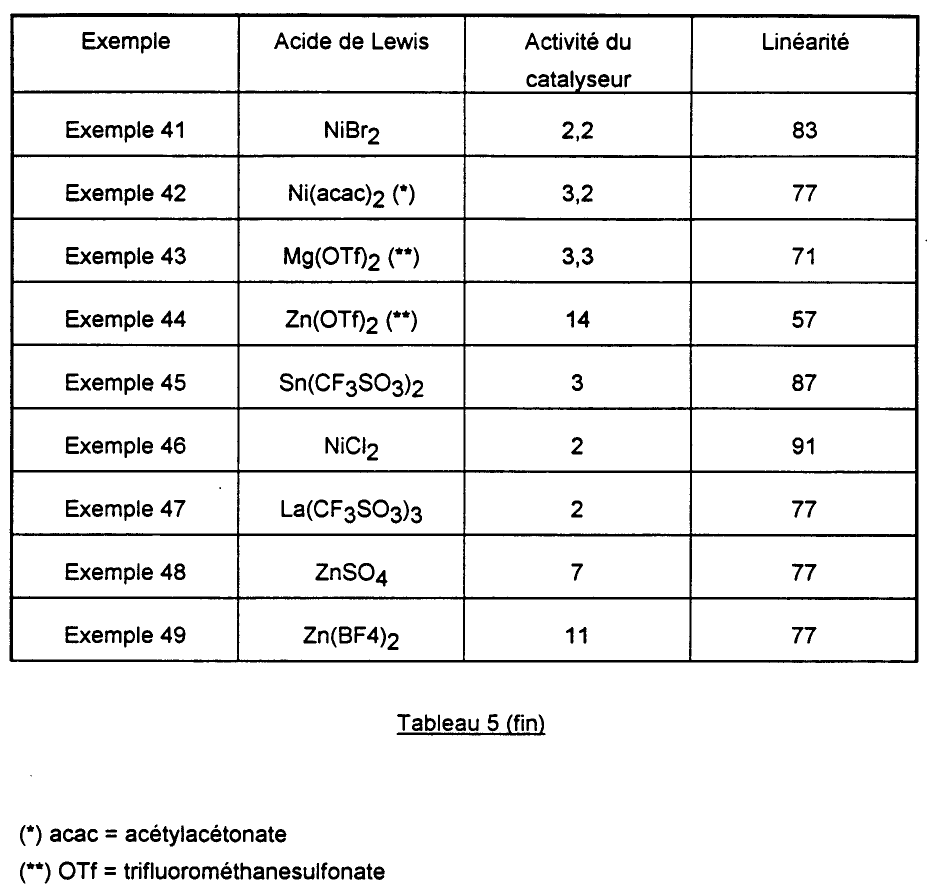

## Revendications

1. Procédé d'hydrocyanation d'un nitrile aliphatique à insaturation éthylénique, par réaction avec le cyanure d'hydrogène, en présence d'une solution aqueuse d'un catalyseur comprenant un composé d'un métal de transition et une phosphine sulfonée, caractérisé en ce que ladite solution contient également un cocatalyseur consistant en au moins un acide de Lewis.

2. Procédé selon la revendication 1, caractérisé en ce que le nitrile à insaturation éthylénique est choisi parmi les pentène-nitriles linéaires comme le pentène-3 nitrile, le pentène-4 nitrile et leurs mélanges.

3. Procédé selon l'une des revendications 1 ou 2, caractérisé en ce que l'on utilise comme composés des métaux de transition des composés du nickel, du palladium et du fer, solubles dans l'eau ou capables de passer en solution dans les conditions de la réaction, et de préférence les composés du nickel.

4. Procédé selon la revendication 3, caractérisé en ce que le composé du nickel utilisé est un composé de nickel zéro.

5. Procédé selon la revendication 3, caractérisé en ce que le composé du nickel utilisé est un composé de nickel à un degré d'oxydation supérieur à zéro.

6. Procédé selon la revendication 5, caractérisé en ce que l'on ajoute au milieu réactionnel un réducteur du nickel, organique ou minéral, réagissant préférentiellement avec le nickel dans les conditions réactionnelles.

7. Procédé selon la revendication 6, caractérisé en ce que le réducteur est ajouté en quantité telle que le nombre d'équivalents d'oxydo-réduction est compris entre 1 et 10.

8. Procédé selon l'une des revendications 1 à 7, caractérisé en ce que la quantité de composé du nickel utilisée est choisie de telle sorte qu'il y ait par litre de solution réactionnelle entre 10⁻⁴ et 1, et de préférence entre 0,005 et 0,5 mole de nickel.

9. Procédé selon l'une des revendications 1 à 8, caractérisé en ce que l'acide de Lewis utilisé comme cocatalyseur est choisi parmi les composés des éléments des groupes Ib, IIb, IIIa, IIIb, IVa, IVb, Va, Vb, VIb, VIIb et VIII de la Classification périodique des éléments, dans la mesure où lesdits composés sont au moins partiellement solubles et stables dans l'eau ou plus généralement dans la phase aqueuse du mélange réactionnel.

10. Procédé selon la revendication 9, caractérisé en ce que les composés sont des sels, tels que des halogénures, de préférence chlorures et bromures, des sulfates, des nitrates, des sulfonates, notamment des trifluorométhanesulfonates, des carboxylates, des acétylacétonates, des tétrafluoroborates et des phosphates.

11. Procédé selon l'une des revendications 1 à 10, caractérisé en ce que l'acide de Lewis est choisi parmi le chlorure de zinc, le bromure de zinc, l'iodure de zinc, le trifluorométhanesulfonate de zinc, I'acétate de zinc, le nitrate de zinc, le tétrafluoroborate de zinc, le chlorure de manganèse, le bromure de manganèse, le chlorure de nickel, le bromure de nickel, le cyanure de nickel, I'acétylacétonate de nickel, le chlorure de cadmium, le bromure de cadmium, le chlorure stanneux, le bromure stanneux, le sulfate stanneux, le tartrate stanneux, les chlorures, bromures, sulfates, nitrates, carboxylates ou trifluorométhanesulfonates des éléments des terres rares comme le lanthane, le cérium, le praséodyme, le néodyme, le samarium, l'europium, le gadolinium, le terbium, le dysprosium, l'holmium, l'erbium, le thulium, l'ytterbium et le lutétium, le chlorure de cobalt, le chlorure ferreux, le chlorure d'yttrium et les mélanges de plusieurs de ces composés.

12. Procédé selon l'une des revendications 1 à 11, caractérisé en ce que l'acide de Lewis est stabilisé en solution aqueuse par l'adjonction d'un chlorure de métal alcalin comme le chlorure de lithium ou le chlorure de sodium.

13. Procédé selon l'une des revendications 1 à 12, caractérisé en ce que le rapport molaire chlorure de lithium ou de sodium/acide de Lewis varie de 0 à 100, le rapport particulier pouvant être ajusté selon la stabilité dans l'eau de l'acide de Lewis.

14. Procédé selon l'une des revendications 1 à 13, caractérisé en ce que le cocatalyseur acide de Lewis mis en oeuvre représente de 0,01 à 50 moles par mole de composé de métal de transition, plus particulièrement de composé du nickel, et de préférence de 1 à 10 moles par mole.

15. Procédé selon l'une des revendications 1 à 14, caractérisé en ce que les phosphines sulfonées mises en oeuvre sont choisies parmi les phosphines sulfonées de formule générale (I) : 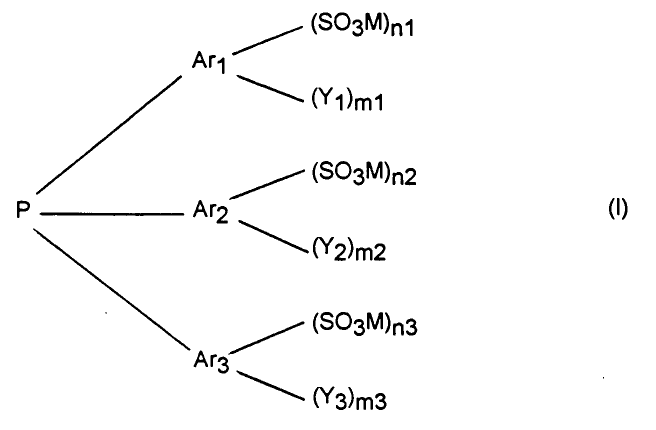 dans laquelle :
* Ar₁, Ar₂ et Ar₃, identiques ou différents, représentent des groupements aryles
* Y₁, Y₂ et Y₃, identiques ou différents, représentent
. un radical alkyle ayant 1 à 4 atomes de carbone,
. un radical alcoxy ayant 1 à 4 atomes de carbone,
. un atome d'halogène,
. un radical CN,
. un radical NO₂
. un radical OH,
. un radical NR₁R₂, dans la formule duquel R₁ et R₂, identiques ou différents, représentent un radical alkyle ayant 1 à 4 atomes de carbone,
* M est un reste cationique minéral ou organique choisi, de manière à ce que le composé de formule (I) soit soluble dans l'eau, dans le groupe comprenant :
. H⁺,
. les cations dérivés des métaux alcalins ou alcalino-terreux,
. N(R₃R₄R₅R₆)⁺ avec R₃,R₄,R₅ et R₆, identiques ou différents, représentant un radical alkyle ayant 1 à 4 atomes de carbone ou un atome d'hydrogène,
. les autres cations dérivés des métaux dont les sels de l'acide benzènesulfonique sont solubles dans l'eau,
* m₁, m₂ et m₃ sont des nombres entiers, identiques ou différents, de 0 à 5,
* n₁, n₂ et n₃ sont des nombres entiers, identiques ou différents, de 0 à 3, l'un d'entre eux au moins étant égal ou supérieur à 1.

16. Procédé selon la revendication 15, caractérisé en ce que les phosphines sulfonées utilisées sont choisies parmi les phosphines sulfonées de formule (I), dans laquelle :
- Ar₁, Ar₂ et Ar₃ sont des groupements phényle,
- Y₁, Y₂ et Y₃ représentent des groupements choisis parmi
. les radicaux alkyle ayant de 1 à 2 atomes de carbone,
. les radicaux alkoxy ayant de 1 à 2 atomes de C - C₁,
- M représente un cation choisi parmi le groupe comprenant
. H⁺
. les cations dérivés de Na, K, Ca, Ba
. NH₄+
. les cations tétraméthylammonium, tétraéthylammonium, tétrapropylammonium, tétrabutylammonium
- m₁, m₂ et m₃ sont des nombres entiers de 0 à 3
- n₁, n₂ et n₃ sont des nombres entiers de 0 à 3, l'un au moins étant égal ou supérieur à 1.

17. Procédé selon l'une des revendications 1 à 16, caractérisé en ce que les phosphines sulfonées utililsées sont choisies parmi les sels de sodium, de potassium, de calcium, de baryum, d'ammonium, de tétraméthylammonium et de tétraéthylammonium, des mono(sulfophényl) diphényl-phosphine, di(sulfophényl) phényl-phosphine et tri(sulfophényl)-phosphine dans les formules desquelles les groupements SO₃ sont de préférence en position méta.

18. Procédé selon l'une des revendications 1 à 17, caractérisé en ce que la quantité de phosphine sulfonée utilisée est choisie de telle sorte que le nombre de moles de ce composé rapporté à 1 mole de métal de transition soit de 0,5 à 2000 et de préférence de 2 à 300.

19. Procédé selon l'une des revendications 1 à 18, caractérisé en ce que la réaction d'hydrocyanation est réalisée à une température de 10°C à 150°C et de préférence de 30°C à 120°C.

## Claims

1. Process for the hydrocyanation of an aliphatic nitrile containing ethylenic unsaturation, by reaction with hydrogen cyanide in the presence of an aqueous solution of a catalyst comprising a transition metal compound and a sulphonated phosphine,
characterized in that the said solution also contains a cocatalyst consisting of at least one Lewis acid.

2. Process according to Claim 1, characterized in that the nitrile containing ethylenic unsaturation is chosen from linear pentenenitriles, for instance 3-pentenenitrile, 4-pentenenitrile and their mixtures.

3. Process according to either of Claims 1 and 2, characterized in that compounds of nickel, of palladium and of iron, which are water-soluble or capable of dissolving under the reaction conditions, and preferably nickel compounds, are used as transition metal compounds.

4. Process according to Claim 3, characterized in that the nickel compound used is a nickel zero compound.

5. Process according to Claim 3, characterized in that the nickel compound used is a compound of nickel with an oxidation state greater than zero.

6. Process according to Claim 5, characterized in that an organic or inorganic nickel-reducing agent which reacts preferentially with nickel under the reaction conditions is added to the reaction medium.

7. Process according to Claim 6, characterized in that the reducing agent is added in an amount such that the number of redox equivalents is between 1 and 10.

8. Process according to one of Claims 1 to 7, characterized in that the amount of nickel compound used is chosen such that there is between 10⁻⁴ and 1 and preferably between 0.005 and 0.5 mol of nickel per litre of reaction solution.

9. Process according to one of Claims 1 to 8, characterized in that the Lewis acid used as cocatalyst is chosen from compounds of the elements of groups Ib, IIb, IIIa, IIIb, IVa, IVb, Va, Vb, VIb, VIIb and VIII of the Periodic Classification of the Elements, insofar as the said compounds are at least partially soluble and stable in water or more generally in the aqueous phase of the reaction mixture.

10. Process according to Claim 9, characterized in that the compounds are salts, in particular halides, preferably chlorides and bromides, sulphates, nitrates, sulphonates in particular trifluoromethanesulphonates, carboxylates, acetylacetonates, tetrafluoroborates and phosphates.

11. Process according to one of Claims 1 to 10, characterized in that the Lewis acid is chosen from zinc chloride, zinc bromide, zinc iodide, zinc trifluoromethane-sulphonate, zinc acetate, zinc nitrate, zinc tetrafluoroborate, manganese chloride, manganese bromide, nickel chloride, nickel bromide, nickel cyanide, nickel acetylacetonate, cadmium chloride, cadmium bromide, stannous chloride, stannous bromide, stannous sulphate, stannous tartrate, chlorides, bromides, sulphates, nitrates, carboxylates or trifluoromethanesulphonates, of rare-earth metal elements, for instance lanthanum, cerium, praseodymium, neodymium, samarium, europium, gadolinium, terbium, dysprosium, holmium, erbium, thulium, ytterbium and lutetium, cobalt chloride, ferrous chloride and yttrium chloride and mixtures of several of these compounds.

12. Process according to one of Claims 1 to 11, characterized in that the Lewis acid is stabilized in aqueous solution by addition of an alkali metal chloride, for instance lithium chloride or sodium chloride.

13. Process according to one of Claims 1 to 12, characterized in that the lithium chloride or sodium chloride/Lewis acid molar ratio varies from 0 to 100, it being possible for the specific ratio to be adjusted depending on the stability in water of the Lewis acid.

14. Process according to one of Claims 1 to 13, characterized in that the Lewis acid cocatalyst used represents from 0.01 to 50 mol per mole of transition metal compound, more particularly of nickel compound, and preferably from 1 to 10 mol per mole.

15. Process according to one of Claims 1 to 14, characterized in that the sulphonated phosphines used are chosen from the sulphonated phosphines of general formula (I): 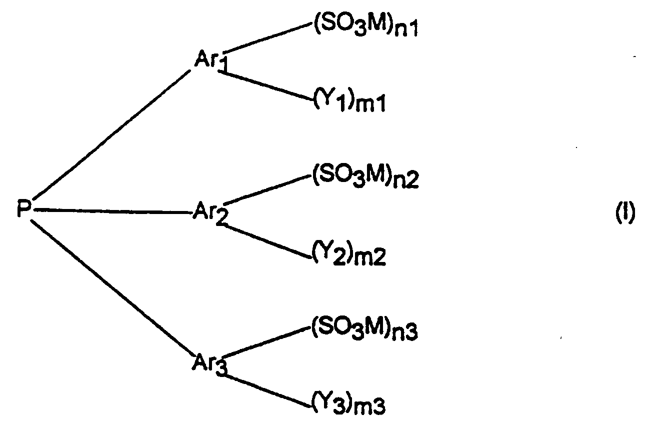 in which:
* Ar₁, Ar₂ and Ar₃, which may be identical or different, represent aryl groups,
* Y₁, Y₂ and Y₃, which may be identical or different, represent
• an alkyl radical having 1 to 4 carbon atoms,
• an alkoxy radical having 1 to 4 carbon atoms,
• a halogen atom,
• a CN radical,
• an NO₂ radical
• an OH radical,
• a radical NR₁R₂ in the formula for which R₁ and R₂, which may be identical or different, represent an alkyl radical having 1 to 4 carbon atoms,
* M is an inorganic or organic cationic residue chosen, so that the compound of formula (I) is water-soluble, from the group comprising:
• H⁺,
• cations derived from alkali metals or alkaline-earth metals,
• N(R₃R₄R₅R₆)⁺ with R₃, R₄, R₅ and R₆, which may be identical or different, representing an alkyl radical having 1 to 4 carbon atoms or a hydrogen atom,
• other cations derived from metals for which the benzenesulphonic acid salts are water-soluble,
* m₁, m₂ and m₃ are integers, which may be identical or different, from 0 to 5,
* n₁, n₂ and n₃ are integers, which may be identical or different, from 0 to 3, at least one of these being greater than or equal to 1.

16. Process according to Claim 15, characterized in that the sulphonated phosphines used are chosen from the sulphonated phosphines of formula (I), in which:
- Ar₁, Ar₂ and Ar₃ are phenyl groups,
- Y₁, Y₂ and Y₃ represent groups chosen from
• alkyl radicals having from 1 to 2 carbon atoms,
• alkoxy radicals having from 1 to 2 carbon atoms,
- M represents a cation chosen from the group comprising
• H⁺
• cations derived from Na, K, Ca and Ba
• NH₄⁺
• tetramethylammonium, tetraethylammonium, tetrapropylammonium and tetrabutylammonium cations
- m₁, m₂ and m₃ are integers from 0 to 3
- n₁, n₂ and n₃ are integers from 0 to 3, at least one being greater than or equal to 1.

17. Process according to one of Claims 1 to 16, characterized in that the sulphonated phosphines used are chosen from the sodium, potassium, calcium, barium, ammonium, tetramethylammonium and tetraethylammonium salts of mono(sulphophenyl)diphenylphosphine, di(sulphophenyl)phenylphosphine and tri(sulphophenyl)phosphine in the formulae for which SO₃ groups are preferably in the meta-position.

18. Process according to one of Claims 1 to 17, characterized in that the amount of sulphonated phosphine used is chosen such that the number of moles of this compound relative to 1 mol of transition metal is from 0.5 to 2000 and preferably from 2 to 300.

19. Process according to one of Claims 1 to 18, characterized in that the hydrocyanation reaction is performed at a temperature of 10°C to 150°C and preferably of 30°C to 120°C.

## Patentansprüche

1. Verfahren zur Hydrocyanierung eines aliphatischen Nitrils mit ethylenischer Unsättigung durch Umsetzung mit Cyanwasserstoff in Anwesenheit einer wäßrigen Lösung eines Katalysators, der eine Übergangsmetallverbindung und ein sulfoniertes Phosphin umfaßt, dadurch gekennzeichnet, daß die Lösung auch einen Cokatalysator enthält, der aus mindestens einer Lewis-Säure besteht.

2. Verfahren nach Anspruch 1, dadurch gekennzeichnet, daß das Nitril mit ethylenischer Unsättigung aus den linearen Pentennitrilen, wie 3-Pentennitril, 4-Pentennitril und deren Mischungen, ausgewählt ist.

3. Verfahren nach einem der Ansprüche 1 oder 2, dadurch gekennzeichnet, daß man als Übergangsmetallverbindungen Verbindungen von Nickel, Palladium und Eisen, die in Wasser löslich sind oder unter den Reaktionsbedingungen in Lösung gehen können, und vorzugsweise Verbindungen von Nickel verwendet.

4. Verfahren nach Anspruch 3, dadurch gekennzeichnet, daß die verwendete Verbindung von Nickel eine Verbindung von Nickel(0) ist.

5. Verfahren nach Anspruch 3, dadurch gekennzeichnet, daß die verwendete Verbindung von Nickel eine Verbindung von Nickel mit einer Oxidationsstufe oberhalb von null ist.

6. Verfahren nach Anspruch 5, dadurch gekennzeichnet, daß man dem Reaktionsmedium ein organisches oder mineralisches Reduktionsmittel für Nickel zusetzt, das unter den Reaktionsbedingungen bevorzugt mit Nickel reagiert.

7. Verfahren nach Anspruch 6, dadurch gekennzeichnet, daß das Reduktionsmittel in einer solchen Menge zugesetzt wird, daß die Zahl der Redox-Äquivalente zwischen 1 und 10 eingeschlossen ist.

8. Verfahren nach einem der Ansprüche 1 bis 7, dadurch gekennzeichnet, daß die verwendete Menge der Nickelverbindung so gewählt ist, daß pro Liter Reaktionslösung zwischen 10⁻⁴ und 1 und vorzugsweise zwischen 0,005 und 0,5 Mol Nickel vorliegen.

9. Verfahren nach einem der Ansprüche 1 bis 8, dadurch gekennzeichnet, daß die als Cokatalysator verwendete Lewis-Säure aus Verbindungen der Elemente der Gruppen Ib, IIb, IIIa, IIIb, IVa, IVb, Va, Vb, VIb, VIIb und VIII des Periodensystems ausgewählt ist, mit der Maßgabe, daß die Verbindungen mindestens teilweise in Wasser oder allgemeiner in der wäßrigen Phase der Reaktionsmischung löslich und stabil sind.

10. Verfahren nach Anspruch 9, dadurch gekennzeichnet, daß die Verbindungen Salze sind, wie Halogenide, vorzugsweise Chloride und Bromide, Sulfate, Nitrate, Sulfonate, insbesondere Trifluormethansulfonate, Carboxylate, Acetylacetonate, Tetrafluoroborate und Phosphate.

11. Verfahren nach einem der Ansprüche 1 bis 10, dadurch gekennzeichnet, daß die Lewis-Säure ausgewählt ist aus Zinkchlorid, Zinkbromid, Zinkiodid, Zinktrifluormethansulfonat, Zinkacetat, Zinknitrat, Zinktetrafluoroborat, Manganchlorid, Manganbromid, Nickelchlorid, Nickelbromid, Nickelcyanid, Nickelacetylacetonat, Cadmiumchlorid, Cadmiumbromid, Zinn(II)chlorid, Zinn(II)bromid, Zinn(II)sulfat, Zinn(II)tartrat, den Chloriden , Bromiden, Sulfaten, Nitraten, Carboxylaten oder Trifluormethansulfonaten der Seltenerdmetalle, wie Lanthan, Cer, Praseodym, Neodym, Samarium, Europium, Gadolinium, Terbium, Dysprosium, Holmium, Erbium, Thulium, Ytterbium und Lutetium, Cobaltchlorid, Eisen(II)chlorid, Yttriumchlorid und Mischungen von mehreren dieser Verbindungen.

12. Verfahren nach einem der Ansprüche 1 bis 11, dadurch gekennzeichnet, daß die Lewis-Säure in wäßriger Lösung durch die Hinzufügung eines Alkalimetallchlorids, wie Lithiumchlorid oder Natriumchlorid, stabilisiert wird.

13. Verfahren nach einem der Ansprüche 1 bis 12, dadurch gekennzeichnet, daß das Molverhältnis Lithium- oder Natriumchlorid/Lewis-Säure zwischen 0 und 100 variiert, wobei das spezielle Verhältnis gemäß der Stabilität der Lewis-Säure in Wasser angepaßt werden kann.

14. Verfahren nach einem der Ansprüche 1 bis 13, dadurch gekennzeichnet, daß der verwendete Lewis-Säure-Cokatalysator 0,01 bis 50 Mol pro Mol Übergangsmetallverbindung, spezieller Nickelverbindung, und vorzugsweise 1 bis 10 Mol pro Mol repräsentiert.

15. Verfahren nach einem der Ansprüche 1 bis 14, dadurch gekennzeichnet, daß die verwendeten sulfonierten Phosphine ausgewählt sind aus den sulfonierten Phosphinen der allgemeinen Formel (I): 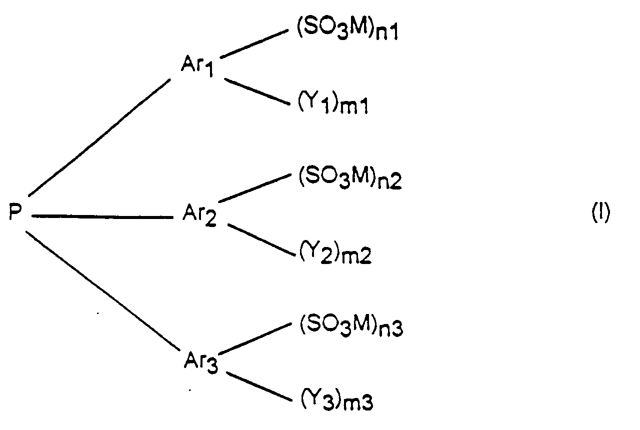 in der:
Ar₁, Ar₂ und Ar₃, identisch oder verschieden, Arylgruppen darstellen,
Y₁, Y₂ und Y₃, identisch oder verschieden,
- einen Alkylrest mit 1 bis 4 Kohlenstoffatomen,
- einen Alkoxyrest mit 1 bis 4 Kohlenstoffatomen,
- ein Halogenatom,
- einen Rest CN,
- einen Rest NO₂,
- einen Rest OH,
- einen Rest NR₁R₂ darstellen, in dessen Formel R₁ und R₂, identisch oder verschieden, einen Alkylrest mit 1 bis 4 Kohlenstoffatomen darstellen,
M ein mineralischer oder organischer kationischer Rest ist, der so, daß die Verbindung der Formel (I) in Wasser löslich ist, aus der Gruppe ausgewählt ist, die umfaßt:
- H⁺,
- die Kationen, die von Alkali- oder Erdalkalimetallen abstammen,
- N(R₃R₄R₅R₆)⁺, wobei R₃, R₄, R₅ und R₆, identisch oder verschieden, einen Alkylrest mit 1 bis 4 Kohlenstoffatomen oder ein Wasserstoffatom darstellen,
- die anderen Kationen, die von Metallen abstammen, deren Benzolsulfonsäuresalze in Wasser löslich sind,
m₁, m₂ und m₃ identische oder verschiedene ganze Zahlen von 0 bis 5 sind,
n₁, n₂ und n₃ identische oder verschiedene ganze Zahlen von 0 bis 3 sind, wobei mindestens eine derselben gleich oder größer als 1 ist.

16. Verfahren nach Anspruch 15, dadurch gekennzeichnet, daß die verwendeten sulfonierten Phosphine aus den sulfonierten Phosphinen der Formel (I) ausgewählt sind, in der:
Ar₁, Ar₂ und Ar₃ Phenylgruppen sind,
Y₁, Y₂ und Y₃ Gruppen darstellen, die ausgewählt sind aus
- Alkylresten mit 1 bis 2 Kohlenstoffatomen,
- Alkoxyresten mit 1 bis 2 C-C₁-Atomen,
M ein Kation ist, das aus der Gruppe ausgewählt ist, die umfaßt
- H⁺,
- die Kationen, die von Na, K, Ca, Ba abstammen,
- NH₄⁺,
- die Kationen Tetramethylammonium, Tetraethylammonium, Tetrapropylammonium, Tetrabutylammonium,
m₁, m₂ und m₃ ganze Zahlen von 0 bis 3 sind,
n₁, n₂ und n₃ ganze Zahlen von 0 bis 3 sind, wobei mindestens eine gleich oder größer als 1 ist.

17. Verfahren nach einem der Ansprüche 1 bis 16, dadurch gekennzeichnet, daß die verwendeten sulfonierten Phosphine ausgewählt sind aus den Natrium-, Kalium-, Calcium-, Barium-, Ammonium-, Tetramethylammonium- und Tetraethylammoniumsalzen von Mono(sulfophenyl)diphenylphosphin, Di(sulfophenyl)phenylphosphin und Tri(sulfophenyl)phosphin, in deren Formeln die Gruppen SO₃ vorzugsweise in der meta-Stellung vorliegen.

18. Verfahren nach einem der Ansprüche 1 bis 17, dadurch gekennzeichnet, daß die verwendete Menge des sulfonierten Phosphins so gewählt wird, daß die Zahl der Mol dieser Verbindung, bezogen auf 1 Mol Übergangsmetall, 0,5 bis 2000 und vorzugsweise 2 bis 300 beträgt.

19. Verfahren nach einem der Ansprüche 1 bis 18, dadurch gekennzeichnet, daß die Hydrocyanierungsreaktion bei einer Temperatur von 10°C bis 150°C und vorzugsweise von 30°C bis 120°C durchgeführt wird.
